# EUROPEAN PATENT SPECIFICATION

(11) **EP 3 868 865 B1**
(45) Date of publication and mention of the grant of the patent: **10.09.2025**
(21) Application number: 21152502.7
(22) Date of filing: 20.01.2021
(51) Int. Cl.: C12N 1/12, C12P 23/00

(54) **METHOD FOR PRODUCING HAEMATOCOCCUS PLUVIALIS USING BIOMINERALIZATION**
VERFAHREN ZUR HERSTELLUNG VON HAEMATOCOCCUS PLUVIALIS MITHILFE VON BIOMINERALISIERUNG
PROCÉDÉ DE PRODUCTION DE HAEMATOCOCCUS PLUVIALIS À L'AIDE DE LA BIOMINÉRALISATION

(30) Priority: 20.02.2020 KR 20200020846
(43) Date of publication of application: 25.08.2021
(73) Proprietor: Korea University Research and Business Foundation, Seoul 02841 (KR)
(72) Inventor: SIM, Sang Jun, 06276 Seoul (KR); YU, Byung Sun, 31194 Chungcheongnam-do (KR); SUNG, Young Joon, 07324 Seoul (KR); HONG, Min Eui, 13644 Gyeonggi-do (KR)
(74) Representative: Grosse, Felix Christopher

(56) References cited:
- KR-B1- 101 554 536
- PAN-UTAI WANIDA ET AL: "Effect of inducing agents on growth and astaxanthin production in Haematococcus pluvialis : Organic and inorganic", BIOCATALYSIS AND AGRICULTURAL BIOTECHNOLOGY, vol. 12, October 2017 (2017-10-01), pages 152 - 158, XP055813217, ISSN: 1878-8181, DOI: 10.1016/j.bcab.2017.10.004
- NAVDEEP K. DHAMI ET AL: "Biomineralization of calcium carbonates and their engineered applications: a review", FRONTIERS IN MICROBIOLOGY, vol. 4, 29 October 2013 (2013-10-29), XP055562342, DOI: 10.3389/fmicb.2013.00314
- HWANG SUNG-WON ET AL: "Acidic cultivation of Haematococcus pluvialis for improved astaxanthin production in the presence of a lethal fungus", BIORESOURCE TECHNOLOGY, vol. 278, April 2019 (2019-04-01), pages 138 - 144, XP085592242, ISSN: 0960-8524, DOI: 10.1016/J.BIORTECH.2019.01.080
- KANG C D ET AL: "Comparison of heterotrophic and photoautotrophic induction on astaxanthin production by Haematococcus pluvialis", APPLIED MICROBIOLOGY AND BIOTECHNOLOGY, SPRINGER, BERLIN, DE, vol. 68, no. 2, August 2005 (2005-08-01), pages 237 - 241, XP019331906, ISSN: 1432-0614, DOI: 10.1007/S00253-005-1889-2

## Description

### BACKGROUND OF THE INVENTION

### 1. Field of the Invention

The present invention relates to a method for producing *Haematococcus pluvialis* using biomineralization, and more specifically to a technology in which biomass containing a high density of cells and a large amount of astaxanthin is produced based on contamination control through biomineralization during mixotrophic cultivation of *Haematococcus pluvialis.*

### 2. Description of the Related Art

Large-scale greenhouse gas emissions arising from the use of fossil fuels cause the phenomenon of global warming. Under these circumstances, carbon capture and sequestration (CCS) technologies for carbon dioxide reduction are actively being developed around the world. CCS technologies refer to methods in which a large amount of carbon dioxide released from a variety of carbon sources, including thermal power plants, is concentrated, captured, and injected into the underground or seabed to isolate it from the atmosphere. CCS technologies are effective in reducing a large amount of carbon dioxide in a short time, but the problems of unstable storage, difficult location selection, and high installation cost make CCS technologies substantially difficult to realize. Thus, carbon capture and utilization (CCU) technologies for directly utilizing carbon dioxide in industrial applications or converting carbon dioxide into high value-added materials rather than sequestering carbon dioxide have recently received more attention than CCS technologies. In Korea, attempts have also been made to develop processes for converting carbon dioxide into high value-added materials. Particularly, biological processes for carbon dioxide conversion utilizing microalgae, photosynthetic microorganisms, have attracted attention as economical techniques that enable the production of various high value-added materials such as biofuels, bioplastics, and biopharmaceuticals, simultaneously with carbon dioxide reduction. Microalgae, called phytoplanktons, are aquatic unicellular organisms that photosynthesize. Microalgae are of increasing interest as highly potential biomass resources due to their ability to produce energy and industrial materials and reduce greenhouse gases. In the future, the utility value of microalgae is expected to rise in industrial fields, particularly energy, chemical, and environmental fields, for the following reasons.

In the energy field, microalgae have the highest oil productivity among all biodiesel producing crops.

Soybean, canola, sunflower, oil palm, etc. have cultivation cycles of 4-8 months whereas microalgae can be cultivated on a daily basis due to their ability to proliferate several times in a day. The annual oil production from microalgae is at least 100 times that from soybean because microalgae have a higher fat content per unit weight. In addition, microalgae are bioresources free from the criticism that food resources are converted into energy and can produce biofuels with similar physical properties to petroleum diesel.

In the chemical field, microalgae can produce numerous valuable substances. Microalgae have been industrialized mainly in the field of food at present and will be industrialized in a wide range of applications such as biochemicals and bioplastics in the future.

In the environmental field, microalgae have attracted the greatest attention due to their ability to reduce carbon dioxide, as described above, and their related studies have been continuously conducted all over the world. Microalgae can absorb carbon dioxide in an amount about twice the weight of biomass, which corresponds to a 10-50 times higher absorption efficiency than that of terrestrial plants. In addition, microalgae can be cultivated irrespective of specific soil and water quality. Related companies are extending the trials to utilize microalgae for carbon dioxide reduction and industrial wastewater purification.

Autotrophic, heterotrophic, and mixotrophic modes are used for microalgal cultivation. Cultivation under autotrophic conditions uses fixed carbon dioxide as a carbon source and light energy to synthesize organic compounds. This mode of cultivation has the advantage that biomass containing large amounts of lipids can be produced but has the disadvantage of a long period of cultivation. Cultivation under heterotrophic conditions requires an additional carbon source as an energy source. This mode of cultivation has the advantage of a short period of cultivation, indicating high productivity, but has a limitation in obtaining high amounts of high value-added materials and is susceptible to contamination such as bacterial and fungal contamination. Mixotrophic conditions refer to a combination of autotrophic and heterotrophic conditions. Cultivation under mixotrophic conditions is advantageous for the production of biomass containing high amounts of high value-added materials in a short period of time but is vulnerable to contamination, limiting its scale-up. These modes of cultivation are adopted depending on the conditions of microalgae but suffer from low rates of carbon dioxide reduction per unit area because of the characteristics of the bioprocesses. Therefore, the carbon dioxide reduction capacity in microalgae-based biomass production processes is an important consideration for companies that release large amounts of greenhouse gases and should treat carbon dioxide emissions.

Microalgae impart economic value to carbon dioxide reduction processes due to their ability to produce high value-added products. High value-added products produced from microalgae are extracted and separated in downstream processes and can be used in a wide range of commercial applications, including pharmaceuticals, health functional foods, and cosmetics. Astaxanthin is a carotenoid that can be extracted from *Haematococcus pluvialis,* a microalgal species. Particularly, astaxanthin has high antioxidant activity and can be used in humans due to its bioactive (3S,3S')-form. Astaxanthin is also very advantageous from an economic standpoint. Thus, much research on biological properties and cultivation processes of *Haematococcus pluvialis* and downstream processes for astaxanthin production has been conducted to maximize the yield of astaxanthin from *Haematococcus pluvialis.*

A method of cultivation of *Haematococcus pluvialis* is described, for example, by Pan-utai Wanida, et al. in 'Effect of inducing agents on growth and astaxanthin production in Haematococcus pluvialis: Organic and inorganic', Biocatalysis and Agricultural Biotechnology, vol. 12 (2017), pages 152-58.

However, conventional processes for the cultivation of *Haematococcus pluvialis* have the problem of contamination caused by microorganisms such as viruses and fungi that inevitably coexist during cultivation, resulting in loss of biomass and astaxanthin contents.

Thus, there is an urgent need for an approach to produce high-density pure biomass containing a large amount of astaxanthin based on contamination control during cultivation of Haematococcus pluvialis.

### SUMMARY OF THE INVENTION

The present invention has been made in an effort to solve the problems of the prior art and one aspect of the present invention is to provide a method for producing *Haematococcus pluvialis* in which pure biomass containing a large amount of astaxanthin as well as calcium carbonate can be obtained by introducing mixotrophic conditions for the cultivation of *Haematococcus pluvialis* and removing contamination factors through biomineralization.

A method for producing *Haematococcus pluvialis* according to the present invention includes (a) supplementing a medium with carbon dioxide to form bicarbonate ions (HCO₃⁻), (b) inoculating *Haematococcus pluvialis* into the medium supplemented with the carbon dioxide, (c) adding sodium acetate to the medium inoculated with the *Haematococcus pluvialis,* followed by first photo-culture, and (d) adding calcium ions (Ca²⁺) to the medium added with the sodium acetate to form calcium carbonate (CaCO₃), followed by second photo-culture.

In step (c) of the method, the sodium acetate is added at a concentration of 5 to 15 mM.

In step (d) of the method, the calcium ions are added at a concentration of 8 to 18 mM.

In step (d) of the method, the calcium ions may be added by supplementing the medium with calcium chloride (CaCl₂).

In the method, step (d) is carried out when the density of the *Haematococcus pluvialis* cells is 1.0 g/L or more in step (c).

In the method, at least one microorganism selected from bacteria, fungi, and microalgae other than *Haematococcus pluvialis* may be generated in at least one of steps (a) to (c) and may be killed by encapsulation with the calcium carbonate.

In the method, the zeta potential of the *Haematococcus pluvialis* is from -30 to -34 eV in step (c) and may be from -6 to -10 eV in step (d).

In the method, the calcium carbonate formed on the surface of the *Haematococcus pluvialis* cells may induce an increase in the size of the *Haematococcus pluvialis* and may be separated from the surface of the *Haematococcus pluvialis* cells with increased size.

In step (d) of the method, a basic solution may be added to the medium to maintain the pH of the medium at 7.5 to 8.5.

The method may further include separating the cultivated *Haematococcus pluvialis* and the calcium carbonate after step (d).

The features and advantages of the present invention will become apparent from the following description with reference to the accompanying drawings.

Prior to the detailed description of the invention, it should be understood that the terms and words used in the specification and the claims are not to be construed as having common and dictionary meanings but are construed as having meanings and concepts corresponding to the technical spirit of the present invention in view of the principle that the inventor can define properly the concept of the terms and words in order to describe his/her invention with the best method.

Conventional methods for the cultivation of *Haematococcus pluvialis* have continued to be commercially unsuccessful due to contamination problems. This failure is overcome by the method of the present invention using biomineralization that enables the mass production of pure biomass containing a large amount of astaxanthin and can efficiently reduce carbon dioxide compared to conventional photo-culture methods.

In addition, according to the method of the present invention, calcium carbonate formed by biomineralization can be separated from biomass. Therefore, further economic effects are expected from the utilization of the separated calcium carbonate.

### BRIEF DESCRIPTION OF THE DRAWINGS

These and/or other aspects and advantages of the invention will become apparent and more readily appreciated from the following description of the embodiments, taken in conjunction with the accompanying drawings of which:
- Figs. 1 and 2: are a flowchart and a diagram illustrating a method for producing *Haematococcus pluvialis* according to one embodiment of the present invention, respectively;
- Fig. 3: is a schematic diagram illustrating biomineralization in a method for producing *Haematococcus pluvialis* according to one embodiment of the present invention;
- Fig. 4: shows the yields (g/L) of *Haematococcus pluvialis* biomass when sodium acetate was added at different concentrations in the growth stage;
- Figs. 5 and 6: show the yields (g/L) of *Haematococcus pluvialis* biomass when calcium chloride was added at different concentrations in the growth stage;
- Fig. 7: shows the yields (g/L) of *Haematococcus pluvialis* biomass when calcium chloride was added at different concentrations in the growth stage after addition of sodium acetate at 10 mM in the growth stage;
- Fig. 8: shows the accumulations (g/L) and contents (%) of astaxanthin when calcium chloride was added at different concentrations in the growth stage after addition of sodium acetate at 10 mM in the growth stage;
- Fig. 9: shows the yields (g/L) of *Haematococcus pluvialis* biomass when sodium acetate and different concentrations of calcium chloride were added under an airlift mode where carbon dioxide was directly added to graduated cylinders;
- Fig. 10: shows the accumulations (g/L) and contents (%) of astaxanthin when sodium acetate and different concentrations of calcium chloride were added under an airlift mode where carbon dioxide was directly added to graduated cylinders;
- Fig. 11: shows the yields of biomass and the accumulations and contents of astaxanthin when contamination factors were directly added to *Haematococcus pluvialis* cells under autotrophic and mixotrophic conditions in the growth stage and when biomineralization was induced after addition of the contamination factors;
- Fig. 12: shows the yields of biomass and the accumulations and contents of astaxanthin when contamination factors were directly added to *Haematococcus pluvialis* cells under autotrophic and mixotrophic conditions in the induction stage and when biomineralization was induced after addition of the contamination factors;
- Fig. 13: shows images of *Haematococcus pluvialis* contaminated with bacteria, fungi, and brown algae and an image of precipitated calcium carbonate and *Haematococcus pluvialis* biomass produced after the contamination factors were killed by biomineralization;
- Fig. 14: is a scanning electron microscopy (SEM) image of a *Haematococcus pluvialis* cell whose surface was covered with contamination factors (fungi);
- Figs. 15 and 16: are scanning electron microscopy (SEM) images of a *Haematococcus pluvialis* cell during biomineralization;
- Fig. 17: is a scanning electron microscopy (SEM) image of a *Haematococcus pluvialis* cell after artificial addition of fungi;
- Fig. 18: is a scanning electron microscopy (SEM) image of the *Haematococcus pluvialis* cell added with fungi shown in Fig. 17 in the induction stage where culture was performed without inducing biomineralization;
- Fig. 19: is a scanning electron microscopy (SEM) image of the *Haematococcus pluvialis* cell added with fungi shown in Fig. 17 in the induction stage where culture was performed while inducing biomineralization;
- Fig. 20: shows scanning electron microscopy (SEM) images showing the progress of biomineralization induced by fungi;
- Fig. 21: shows scanning electron microscopy (SEM) images showing the progress of biomineralization induced by brown algae;
- Fig. 22: shows scanning electron microscopy (SEM) images showing the progress of biomineralization induced by bacteria;
- Fig. 23: shows microscopy (SEM) images and fluorescence intensities showing the progress of biomineralization induced by bacteria;
- Fig. 24: shows a process for separating *Haematococcus pluvialis* biomass and precipitated calcium carbonate using Percoll and scanning electron microscopy (SEM) images of the separated biomass and calcium carbonate;
- Fig. 25: shows graphs showing the yield (g/L) of biomass and the content (%) of astaxanthin after the separated pure *Haematococcus pluvialis* shown in Fig. 24 was cultivated under autotrophic conditions for 15 days; and
- Fig. 26: shows a process for separating *Haematococcus pluvialis* biomass and precipitated calcium carbonate using a large-capacity centrifuge and scanning electron microscopy (SEM) images of the separated biomass and calcium carbonate.

### DETAILED DESCRIPTION OF THE INVENTION

Other objects, advantages, and novel features of the invention will become more apparent from the following detailed description and preferred embodiments with reference to the appended drawings. In the drawings, the same elements are denoted by the same reference numerals even though they are depicted in different drawings. Although such terms as "first" and "second," etc. may be used to describe various elements, these elements should not be limited by above terms. These terms are used only to distinguish one element from another. In the description of the present invention, detailed explanations of related art are omitted when it is deemed that they may unnecessarily obscure the essence of the present invention.

Preferred embodiments of the present invention will now be described in detail with reference to the accompanying drawings.

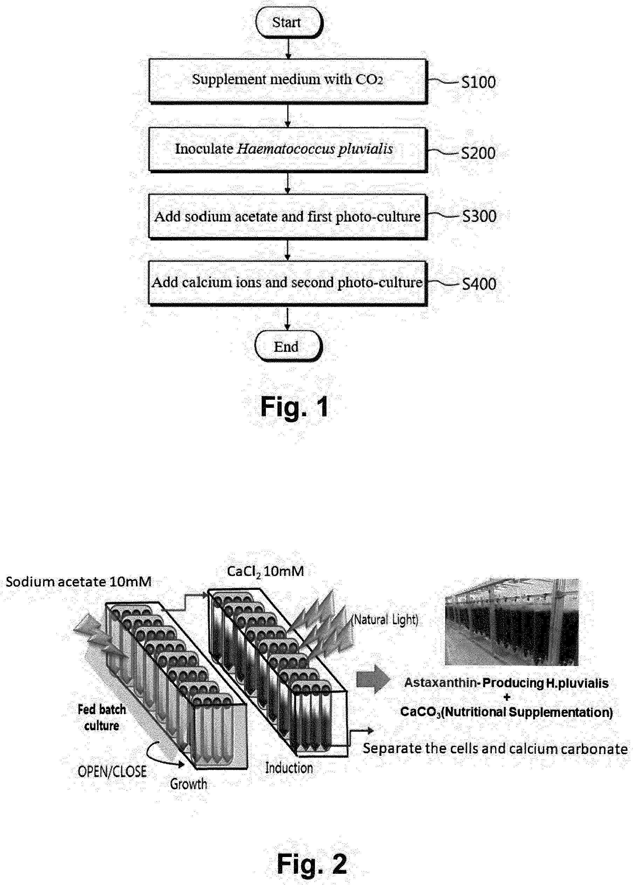

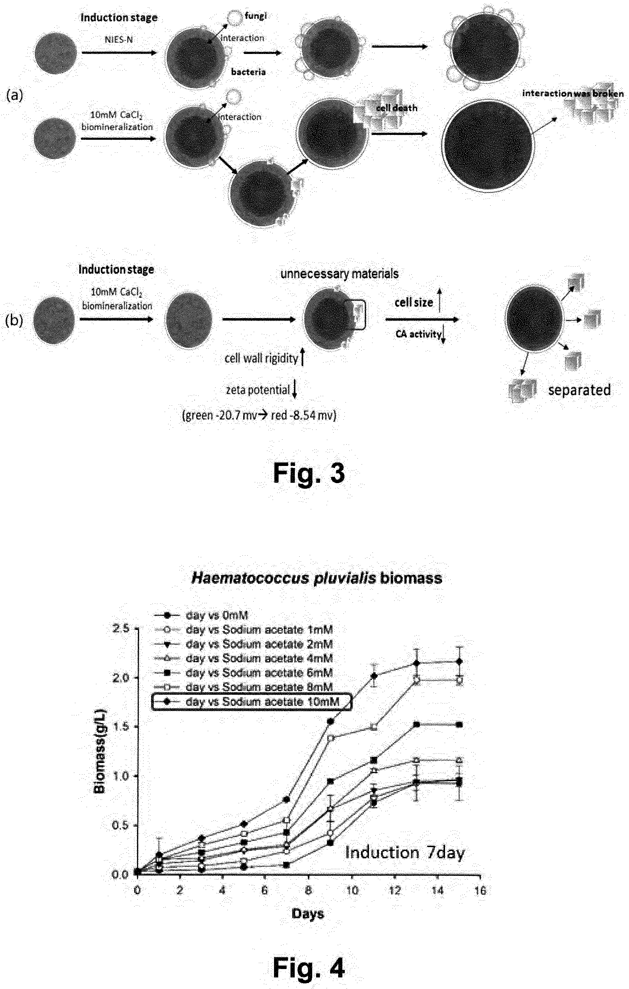

Figs. 1 and 2 are a flowchart and a diagram illustrating a method for producing *Haematococcus pluvialis* according to one embodiment of the present invention, respectively, and Fig. 3 is a schematic diagram illustrating biomineralization in a method for producing *Haematococcus pluvialis* according to one embodiment of the present invention.

As shown in Figs. 1 and 2, the method includes (a) supplementing a medium with carbon dioxide to form bicarbonate ions (HCO₃⁻) (S100), (b) inoculating *Haematococcus pluvialis* into the medium supplemented with the carbon dioxide (S200), (c) adding sodium acetate to the medium inoculated with the *Haematococcus pluvialis,* followed by first photo-culture (S300), and (d) adding calcium ions (Ca²⁺) to the medium added with the sodium acetate to form calcium carbonate (CaCO₃), followed by second photo-culture (S400).

The present invention relates to a method for producing biomass by cultivating *Haematococcus pluvialis,* a microalgal species. With an increasing necessity for greenhouse gas treatment technologies as a result of global warming, research on technologies for bioconversion of carbon dioxide using microalgae has been conducted worldwide. Microalgae impart economic value to carbon dioxide reduction processes due to their ability to produce high value-added products. Particularly, *Haematococcus pluvialis* produces bioactive astaxanthin with high antioxidant activity. *Haematococcus pluvialis* cells are rich in nutrients and tend to divide frequently under low light conditions. *Haematococcus pluvialis* cells are motile flagellated cells. *Haematococcus pluvialis* cells lose their motility and form hard cysts when starved of nutrients and under high light stress. At this time, astaxanthin, a carotenoid with high antioxidant activity, accumulates as a secondary metabolite in the *Haematococcus pluvialis* cells as a defense mechanism against oxidative stress. It is known that the ratio of carotenoid to chlorophyll content is usually about 0.2 in the growth stage but the carotenoid content increases gradually in the induction stage.

In the induction stage, *Haematococcus pluvialis* cells form cysts, contain astaxanthin accumulated therein, and develop cell walls. The cell wall of *Haematococcus pluvialis* cyst cells consists of trilayers: the outermost trilaminar sheath composed of algaenan resistant to acetolysis; the secondary wall composed of mannose and cellulose with homogeneous arrangement; and the tertiary wall composed of mannose and cellulose with heterogeneous arrangement.

*Haematococcus pluvialis* having these characteristics has been cultivated under autotrophic conditions. However, contamination with microorganisms such as viruses, fungi, and other microalgae is inevitable during cultivation under autotrophic conditions, leading to loss of biomass and astaxanthin contents. The present invention has been made in an effort to provide a solution to the problems of the prior art.

Specifically, the method of the present invention includes (S100) supply of carbon dioxide, (S200) inoculation with *Haematococcus pluvialis,* (S300) addition of sodium acetate and first photo-culture, and (S400) addition of calcium ions and second photo-culture.

In S100, a medium is supplemented with carbon dioxide to photo-culture *Haematococcus pluvialis, a* microalgal species. The medium may be filled in a reaction vessel. Any medium that enables the growth of *Haematococcus pluvialis* can be used without particular limitation. For example, the medium may be a carbon-deficient autotrophic medium. The carbon dioxide supply can be accomplished by supplying air containing 4 to 6% (v/v) carbon dioxide or using a CO₂ incubator. Alternatively, exhaust gas may be supplied to the medium. The supplied carbon dioxide reacts with water in the medium to form hydrogen ions and bicarbonate ions (HCO₃⁻). The carbon dioxide may be supplied temporarily or continuously. The carbon dioxide may be continuously supplied until the addition of calcium ions and second photo-culture in the subsequent step (S400).

In S200, *Haematococcus pluvialis* is inoculated into the medium supplemented with the carbon dioxide.

The step of adding sodium acetate and first photo-culture (S300) is carried out to grow the inoculated *Haematococcus pluvialis.* In this step, the *Haematococcus pluvialis* cells grow during the photo-culture. The sodium acetate is added as an energy source. In the method of the present invention, *Haematococcus pluvialis* is cultivated under mixotrophic conditions. Mixotrophic conditions refer to a combination of autotrophic conditions using fixed carbon dioxide as a carbon source and heterotrophic conditions using an additional energy source (sodium acetate). Thus, the method of the present invention enables the mass production of biomass containing a large amount of astaxanthin in a short period of time.

In the growth stage, the sodium acetate may be added at a concentration of 5 to 15 mM to the medium. The concentration of the sodium acetate is preferably 10 mM. This concentration range is effective in increasing the yields of biomass and astaxanthin.

The first photo-culture may be performed by irradiation with light at an intensity of 10 to 30 µE/m²/s. Since nutrients are abundant and low light conditions are maintained in the growth stage, the *Haematococcus pluvialis* cells are motile flagellated cells that divide actively.

In S400, calcium ions are added and second photo-culture is performed. S400 corresponds to the induction stage where the *Haematococcus pluvialis* is photo-cultured. The second photo-culture is performed at a relatively high light intensity compared to the first photo-culture. As an example, the second photo-culture may be performed by irradiation with light at an intensity of 40 to 60 µE/m²/s. In the induction stage, the medium may be continuously supplemented with carbon dioxide, as described above, but sodium acetate is not added thereto. In the induction stage where nutrients are depleted and high light stress is applied, the *Haematococcus pluvialis* cells lose their motility and form hard cysts and the accumulation of astaxanthin in the cells can be induced as a defense mechanism against oxidative stress.

Here, the entry of the *Haematococcus pluvialis* into the induction stage can be determined by observing whether a change in the morphology of the *Haematococcus pluvialis* or nitrogen starvation in the medium occurs. The induction stage can be carried out when the density of the *Haematococcus pluvialis* cells in the medium is 1.0 g/L or more.

One or more microorganisms selected from bacteria, fungi, and microalgae (e.g., brown algae) other than the *Haematococcus pluvialis* exist especially due to the carbon source under mixotrophic conditions in the growth stage, where the sodium acetate is added, before the induction stage. These microorganisms are contamination factors that cause loss of biomass and astaxanthin production. In the method of the present invention, the contamination factors are removed by the addition of calcium ions (Ca²⁺) to induce biomineralization. Microorganisms have carbonic anhydrase (CA), which acts as a catalyst for the reaction of water and carbon dioxide to form hydrogen ions and bicarbonate ions. The bicarbonate ions react with the calcium ions added to the medium to form calcium carbonate (CaCO₃). As shown in (a) of Fig. 3, contamination factors such as fungi adhere to the surface of the *Haematococcus pluvialis* cells in the induction stage to inhibit the growth of the cells (top) and are killed by the addition of calcium carbonate to induce biomineralization (bottom). (b) of Fig. 3 shows the principle that calcium carbonate adherent to the cell surface falls off. Referring to Fig. 3, microorganisms such as fungi, bacteria, and other microalgae proliferate in the induction stage. Specifically, fungi adhere mainly to the surface of the *Haematococcus pluvialis* cells (see the top of (a) of Fig. 3) and bacteria and other microalgae coexist with the *Haematococcus pluvialis* cells in the medium to inhibit the growth of the cells. In the method of the present invention, contamination control is performed by adding calcium ions. Here, the calcium ions and the bicarbonate ions induce biomineralization, i.e. crystallization, and the resulting calcium carbonate crystals encapsulate microorganisms such as fungi. This encapsulation leads to nutrient starvation, eventually resulting in the death of the microorganisms (see the bottom of (a) of Fig. 3). Meanwhile, the *Haematococcus pluvialis* has CA but its death is not caused by biomineralization. This is because the zeta potential of the *Haematococcus pluvialis* is from -30 to -34 eV in the growth stage but is changed to -6 to -10 eV in the induction stage. This change in zeta potential makes it difficult for the calcium ions to bind to the surface of the *Haematococcus pluvialis,* and as a result, calcium carbonate is hardly formed on the surface of the *Haematococcus pluvialis.* Although calcium carbonate is partially formed on the cell surface, the cells recognize the calcium carbonate as a stress factor and tend to thicken their wall. As a result, the cell surface is not stabilized and the calcium carbonate is separated therefrom. That is, the calcium carbonate formed on the cell surface induces an increase in the size of the *Haematococcus pluvialis* and finally falls off from the cell surface (see (b) of Fig. 3). Fungi recognize glycoproteins present in the cell wall of the *Haematococcus pluvialis* and bind to the cell surface. When the calcium carbonate encapsulates and kills the fungi, the bonds between the cells and the fungi are broken and the calcium carbonate is thus separated from the cells. Furthermore, the death of bacteria and other microalgae in the medium increases the photosynthetic efficiency of the *Haematococcus pluvialis* to induce an increase in the size of the *Haematococcus pluvialis,* facilitating the separation of the calcium carbonate bound to the surface of the *Haematococcus pluvialis* cells. The calcium carbonate in the medium reflects light in water, contributing to enhanced photosynthetic efficiency of the *Haematococcus pluvialis.* In conclusion, the biomineralization causes the death of microorganisms other than the *Haematococcus pluvialis,* leading to increases in the production of biomass and the amount of astaxanthin accumulated.

The calcium ions for biomineralization-based contamination control can be provided by supply of calcium chloride (CaCl₂). However, the source of the calcium ions is not necessarily limited to calcium chloride. Any known calcium salt that can be dissolved to supply calcium ions may be used. Examples of such calcium salts include Ca(OH)₂, Ca(NO₃)₂·4H₂O, Ca(CH₃COO)₂·H₂O, and CaSO₄·2H₂O. The calcium ions are added at a concentration of 8 to 18 mM, preferably 10 mM, to the medium.

The addition of the calcium chloride produces calcium carbonate and water, leading to a decrease in the pH of the medium. When dissolved, carbon dioxide continuously produces bicarbonate ions and hydrogen ions, resulting in a pH decrease to about 3 to about 4.5. Since the biomineralization proceeds at a pH of 7.5 to 8.5, preferably 7.7 to 8.0, a basic solution is added to the medium to maintain the pH of the medium at 7.5 to 8.5 in the induction stage. The basic solution may be a solution of at least one base selected from the group consisting of sodium hydroxide (NaOH), potassium hydroxide (KOH), ammonium hydroxide (NH₄OH), calcium hydroxide (Ca(OH)₂), and magnesium hydroxide (Mg(OH)₂).

Astaxanthin accumulates in a large amount in the *Haematococcus pluvialis* cells in the induction stage and can be obtained by separating the *Haematococcus pluvialis* and the calcium carbonate based on their different densities. As an example, a centrifuge may be used to separate the *Haematococcus pluvialis* and the calcium carbonate. The calcium carbonate can be easily separated from the *Haematococcus pluvialis* without addition of any additional chemical because the calcium carbonate exists in a crystal form.

Overall, conventional methods for the cultivation of *Haematococcus pluvialis* have continued to be commercially unsuccessful due to contamination problems. This failure is overcome by the method of the present invention using biomineralization that enables the mass production of pure biomass containing a large amount of astaxanthin and can efficiently reduce carbon dioxide compared to conventional photo-culture methods. In addition, according to the method of the present invention, calcium carbonate formed by biomineralization can be separated from biomass. Therefore, further economic effects are expected from the utilization of the separated calcium carbonate.

The present invention will be explained more specifically with reference to the following experimental examples.

### Experimental Example 1: Experimental setup and method

Twenty-five 250 ml flasks were prepared to find primary optimal conditions. However, since only a limited amount of carbon dioxide can be dissolved in the flasks and carbon dioxide should be introduced in an airlift mode, fourteen 500 ml graduated cylinders (width: 5 cm, height: 60 cm) were prepared after the optimization experiment in the flasks.

Each graduated cylinder was equipped with a versatile silicone stopper capable of closing the opening and through which gas injection and sampling could be done. Two Teflon tubes as gas and sampling lines were inserted into 2-3 mm holes formed in the stopper. The tubes were cut corresponding to the height of the graduated cylinder. A stone sparger for the supply of air containing 5% carbon dioxide was connected to the bottom end of the Teflon tube for gas supply. For the flask experiment, incubation was performed in a 5% CO2 incubator.

NIES-C was used as a medium and HEPES was used as a buffer in the flasks. After 250 ml of the medium was introduced into each of the 250 ml flasks, incubation was performed in the incubator. After 450 ml of the medium was introduced into each of the graduated cylinders, aeration was performed. A 10 mM KOH solution was introduced to adjust the pH to 7.5-8. Due to the buffering effect of the KOH solution, the pH could be maintained between 7.5 and 8 despite a continuous supply of carbon dioxide. Haematococcus pluvialis was used as a microalgal species. The microalgal species was inoculated at an initial density of 0.05 g/L, based on biomass. The flasks and the graduated cylinders were irradiated with light at 300 µE/m2/s for 6-7 days in the growth stage and at 50 µE/m2/s for 7-8 days in the induction stage. The final culture volume of each graduated cylinder was adjusted to 500 ml.

Sampling was conducted at the same time every day. Different methods were needed to measure biomass and calcium carbonate that coexisted in each sample. This problem was solved as follows. First, the medium was removed from each sample (10 ml) using a centrifuge, followed by washing three times with distilled water (pH 8). After additional centrifugation. 5 ml of the precipitate containing both biomass and calcium carbonate was weighed. As for the remainder (5 ml), the supernatant was removed and 35 ml of distilled water whose pH was adjusted to ≤5 with HCl was added. The calcium carbonate was allowed to react with the hydrochloric acid with vortexing for 30-60 sec. As a result of the reaction, CaCl₂ and carbon dioxide were formed. The reaction mixture was centrifuged to remove the supernatant and the precipitate was washed with distilled water (pH 8) to remove calcium carbonate.

### Experimental Example 2: Optimization of sodium acetate and calcium chloride in the growth stage

Simultaneously with inoculation of cells, sodium acetate was added to introduce the advantages of heterotrophic cultivation that enables the production of biomass in a short time. In order to find an optimal sodium acetate concentration, cells were cultured while adding sodium acetate at different concentrations of 1 mM to 10 mM into the flasks, and the yields of biomass were analyzed.

Fig. 4 shows the yields (g/L) of *Haematococcus pluvialis* biomass when sodium acetate was added at different concentrations in the growth stage. Referring to Fig. 4, the highest yield of biomass was obtained when sodium acetate was added at 10 mM. Although not shown in Fig. 4, the addition of sodium acetate at concentrations higher than 10 mM rather inhibited the cell growth. Also when applied to the graduated cylinders, the yield of biomass reached a maximum at 10 mM sodium acetate.

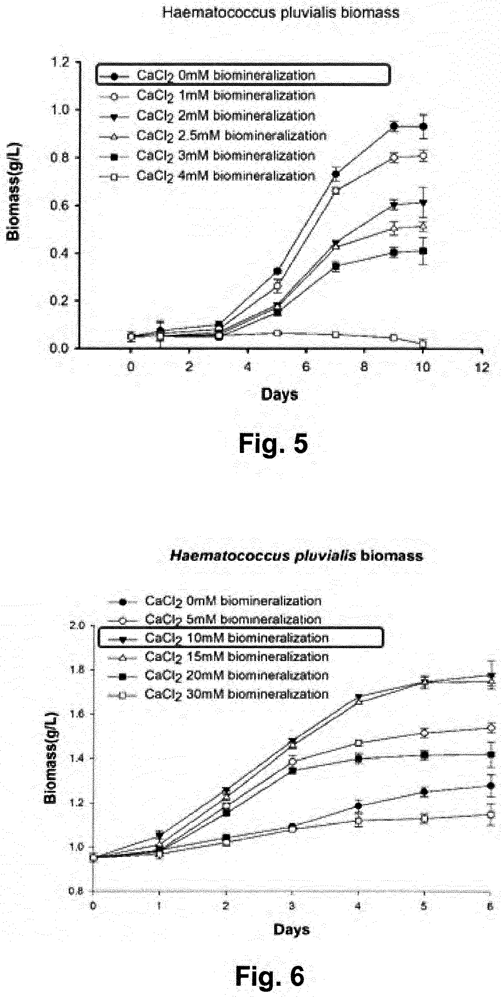

CaCl₂ was added at different concentrations (0-30 mM) for biomineralization in the growth stage and biomass growth was observed. Figs. 5 and 6 show the yields (g/L) of *Haematococcus pluvialis* biomass when calcium chloride was added at different concentrations in the growth stage. Referring to Fig. 5, even the addition of CaCl₂ at 1 mM inhibited biomass growth. Referring to Fig. 6, the highest biomass yield was observed without nitrogen (N)-starvation when CaCl₂ was added at 10 mM in the induction stage. Therefore, the optimal concentration of CaCl₂ added in the induction stage was 10 mM.

### Experimental Example 3: Optimization of calcium chloride in the induction stage

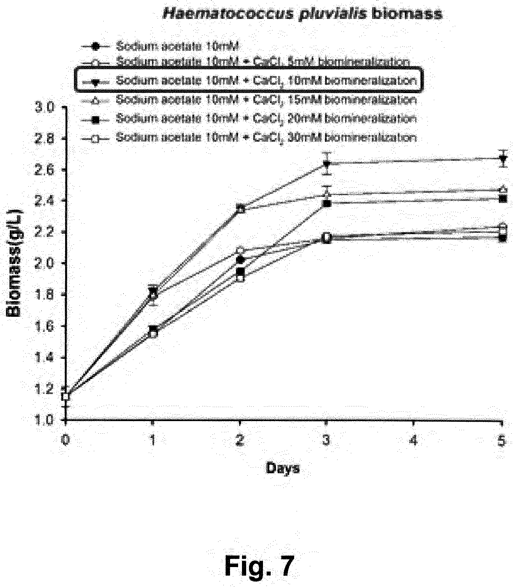

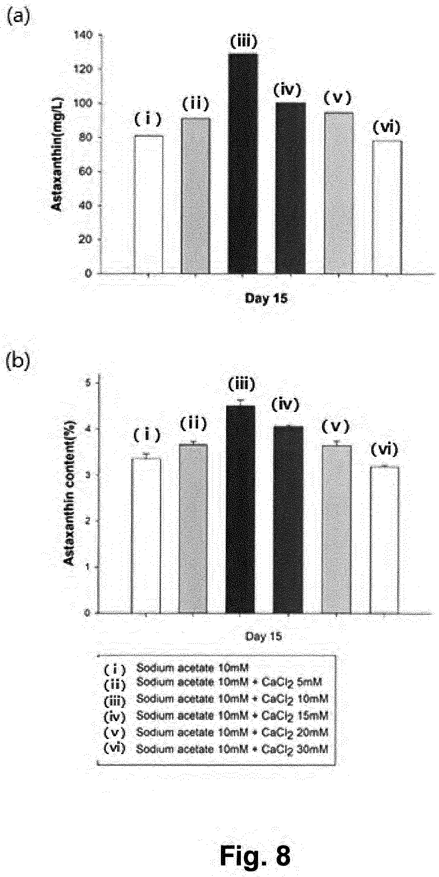

An experiment was conducted to find an optimal concentration of CaCl₂ added to the flasks in the induction stage. Mineralization was conducted by fixing the concentration of sodium acetate in the medium in the growth stage at 10 mM and adding CaCl₂ at various concentrations of 0 to 30 mM when the yield of biomass was 1.1 g/L. At this time, KOH was added to maintain the pH of the medium at 7.7-8.0. The results are shown in Figs. 7 and 8. Fig. 7 shows the yields (g/L) of *Haematococcus pluvialis* biomass when calcium chloride was added at different concentrations in the growth stage after addition of sodium acetate at 10 mM in the growth stage. Fig. 8 shows the accumulations (g/L) and contents (%) of astaxanthin when calcium chloride was added at different concentrations in the growth stage after addition of sodium acetate at 10 mM in the growth stage. As can be seen from Figs. 7 and 8, the highest yield of biomass and the highest accumulation and content of astaxanthin were obtained when the concentration of sodium acetate in the medium was 10 mM in the growth stage and the concentration of CaCl₂ was 10 mM in the induction stage.

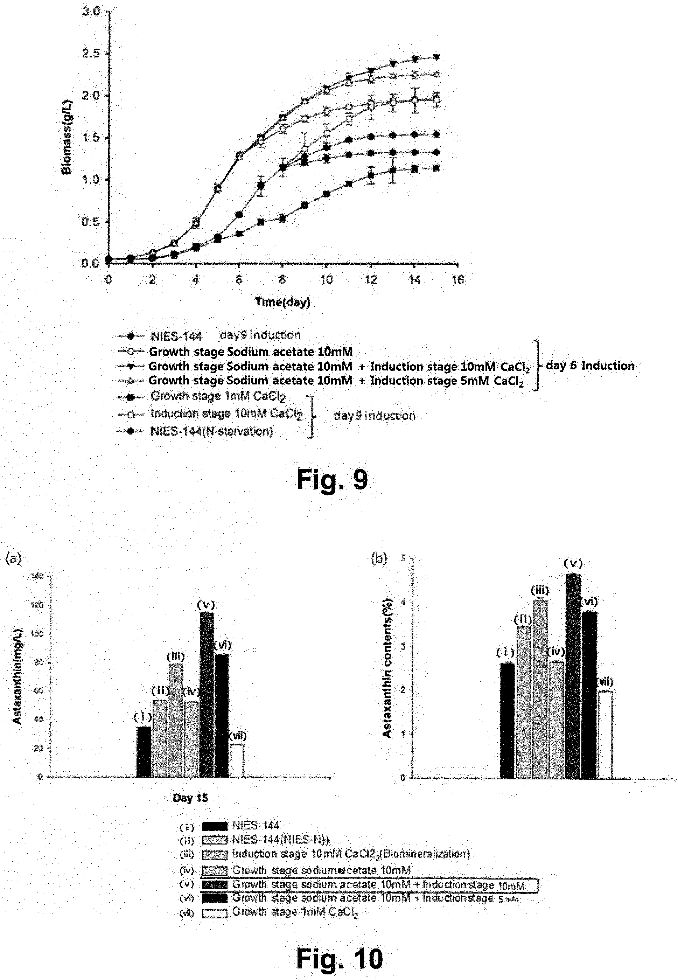

As in the flask experiment, sodium acetate was added at 10 mM in the growth stage and CaCl₂ was added at 1, 5, and 10 mM in the induction stage when the yield of biomass was 1.1 g/L. Thereafter, the contents of biomass and astaxanthin in the graduated cylinders were analyzed and the results are shown in Figs. 9 and 10. Fig. 9 shows the yields (g/L) of *Haematococcus pluvialis* biomass when sodium acetate and different concentrations of calcium chloride were added under an airlift mode where carbon dioxide was directly added to the graduated cylinders. Fig. 10 shows the accumulations (g/L) and contents (%) of astaxanthin when sodium acetate and different concentrations of calcium chloride were added under an airlift mode where carbon dioxide was directly added to the graduated cylinders. The highest yield of biomass and the highest accumulation (see (a) of Fig. 10) and content of astaxanthin (see (b) of Fig. 10) were obtained when the concentration of sodium acetate was 10 mM in the growth stage and the concentration of CaCl₂ was 10 mM in the induction stage. The results are in agreement with the results obtained in the flask experiment.

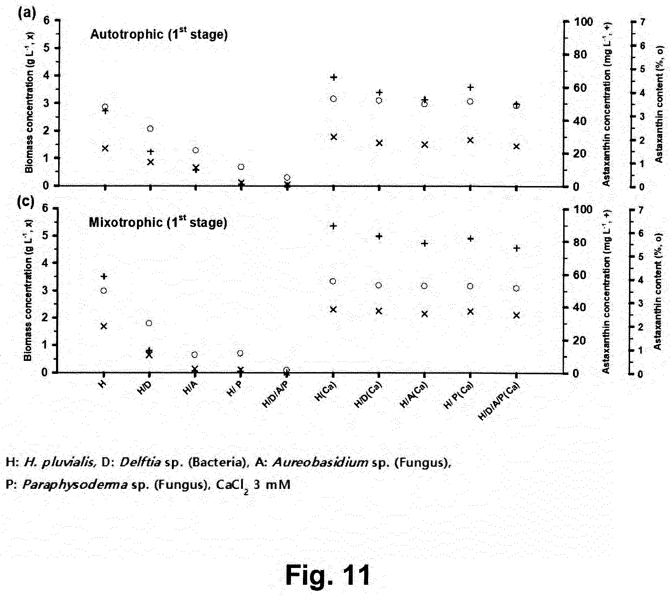

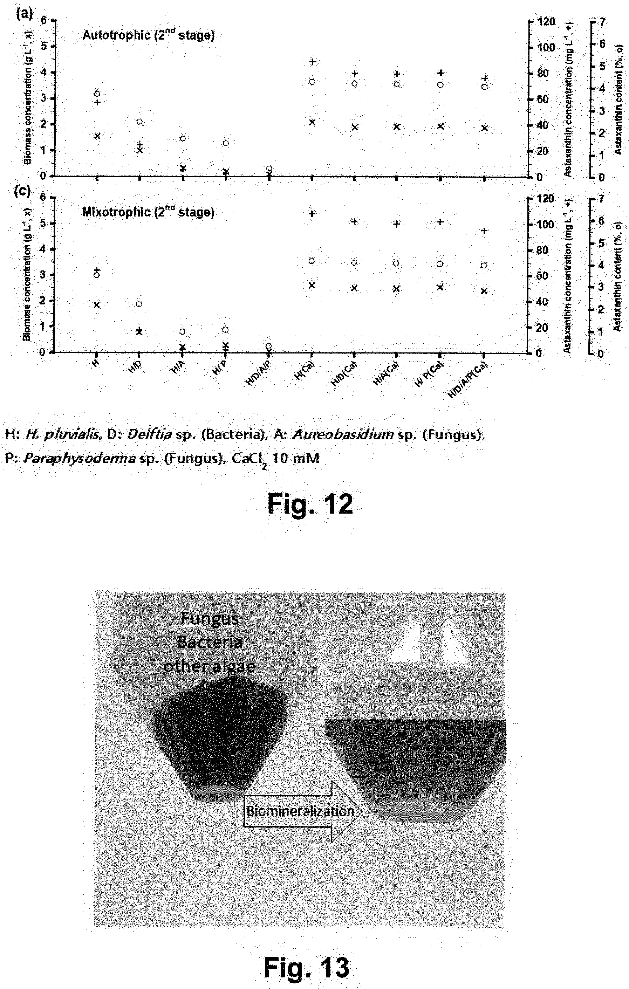

Fungal and bacterial species were found during cultivation. The fungal species were identified as *Aureobasidium pullans* and *Paraphysoderma sedebokerensis.* The bacterial species was identified as *Delftia tsurhatensis.* These species were added to *Haematococcus pluvialis* and biomineralization was performed. First, the contamination factors were added in the growth stage under autotrophic conditions. As can be seen in Figs. 11 and 12, when biomineralization was not performed, significant losses occurred in the yield of biomass and the accumulation and content of astaxanthin. In contrast, few losses were observed when biomineralization was performed. When the contamination factors were added in the growth stage under mixotrophic conditions and biomineralization was performed, few losses were observed (see Fig. 11). Both when the contamination factors were added in the induction stage under autotrophic conditions and biomineralization was performed and when the contamination factors were added in the induction stage under mixotrophic conditions and biomineralization was performed, few losses occurred in the yield of biomass and the accumulation and content of astaxanthin (see Fig. 12). Here, it could be confirmed that when mineralization was performed during pure cultivation of *Haematococcus pluvialis* without any contamination source, the yield of biomass and the accumulation of astaxanthin were improved. Considering cellular stress induced by calcium chloride, mineralization was performed in the presence of calcium chloride at a low concentration of 3 mM in the growth stage. In the induction stage, mineralization was performed in the presence of calcium chloride at 10 mM.

Fig. 13 shows (left) an image of *Haematococcus pluvialis* produced by cultivation without biomineralization and (right) an image of *Haematococcus pluvialis* produced by cultivation with biomineralization. The amount of biomass collected after cultivation under autotrophic conditions (see the left-hand image of Fig. 13) was larger than that collected after cultivation with biomineralization (see the right-hand image of Fig. 13). The content of calcium carbonate in the biomass collected after cultivation with biomineralization was 0.074 g/L.

### Experimental Example 4: Determination of contamination control through biomineralization

In this experimental example, a determination was made as to whether contamination with fungi, bacteria or brown algae was controlled by biomineralization. To this end, sodium acetate was added at 10 mM in the growth stage and CaCl₂ was not added and then CaCl₂ was optionally added at 10 mM in the induction stage, as in Experimental Example 3. After cultivation, centrifugation was performed. Fig. 13 shows images of *Haematococcus pluvialis* contaminated with bacteria, fungi, and brown algae and an image of precipitated calcium carbonate and *Haematococcus pluvialis* biomass produced after the contamination factors were killed by biomineralization. The left-hand image of Fig. 13 reveals that *Haematococcus pluvialis* cells were contaminated with fungi, bacteria, and brown algae in the absence of CaCl₂. In contrast, the right-hand image of Fig. 13 reveals that when CaCl₂ was added, fungi were killed in the supernatant and white calcium carbonate settled down at the bottom. In conclusion, biomineralization produces calcium carbonate and causes nutrient starvation (encapsulation) to kill fungi, bacteria, and other microalgae.

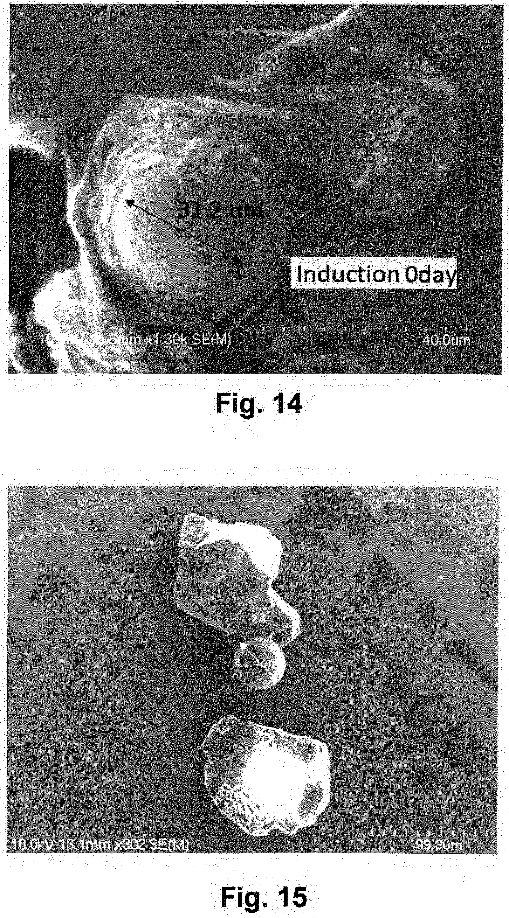

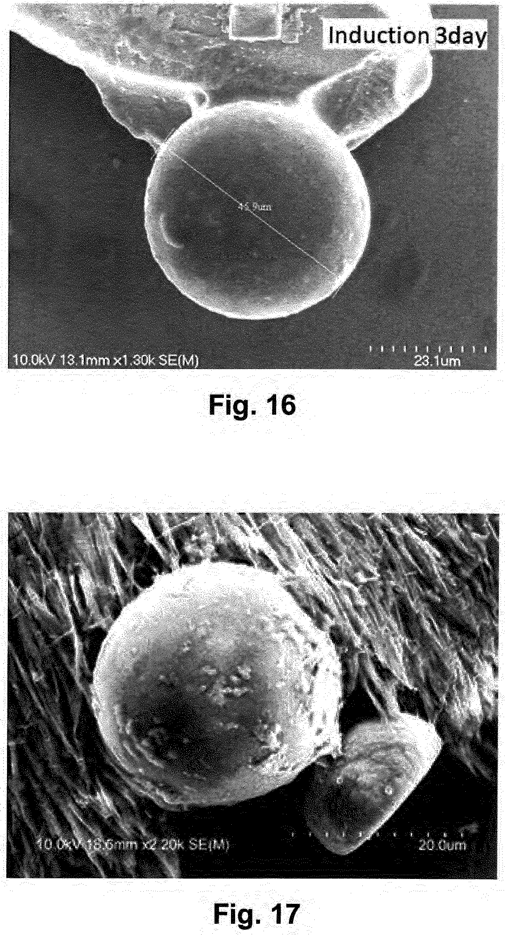

*Haematococcus pluvialis* cells were observed using a scanning electron microscope (SEM) before and after the addition of CaCl₂. Fig. 14 is a scanning electron microscopy (SEM) image of a *Haematococcus pluvialis* cell whose surface was covered with contamination factors (fungi). Figs. 15 and 16 are scanning electron microscopy (SEM) images of a *Haematococcus pluvialis* cell during biomineralization. Referring to Fig. 14, fungi adherent to the surface of the *Haematococcus pluvialis* cell caused contamination before biomineralization. The cell size was 31.2 µm. The image of Fig. 15 was taken 2 days after the addition of CaCl₂. Referring to Fig. 15, the fungi adherent to the surface of the *Haematococcus pluvialis* cell were biomineralized to produce calcium carbonate and fell off from the cell surface. The cell size was 41.4 µm. The image of Fig. 16 was taken 3 days after the addition of CaCl₂. The cell size increased to 45.9 µm, indicating that the removal of the fungi led to an increase in the cell size.

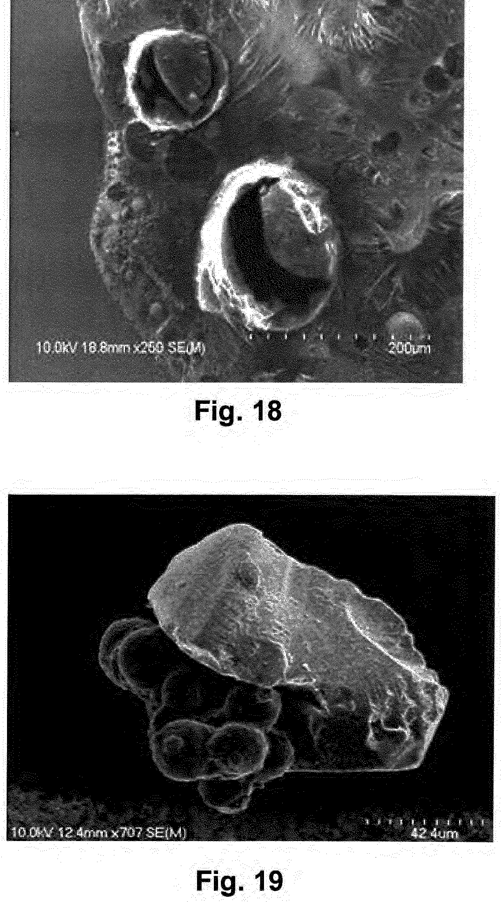

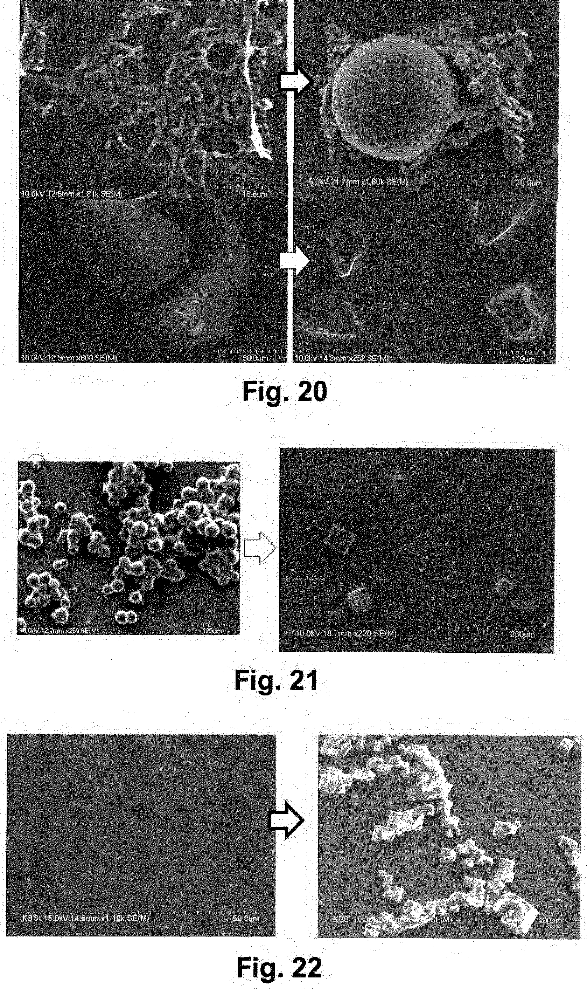

The influence of fungi on *Haematococcus pluvialis* and the crystallization of fungi by biomineralization were investigated. To this end, fungi were artificially added to *Haematococcus pluvialis* cells and observed with a scanning electron microscope (SEM). Fig. 17 is a scanning electron microscopy (SEM) image of a *Haematococcus pluvialis* cell after the artificial addition of fungi. The fungi were observed to be adherent to the surface of the *Haematococcus pluvialis* cell. Fig. 18 is a scanning electron microscopy (SEM) image of the *Haematococcus pluvialis* cell added with fungi shown in Fig. 17 in the induction stage where culture was performed without inducing biomineralization. The fungi were observed to attack the *Haematococcus pluvialis* cell. Figs. 19 and 20 are scanning electron microscopy (SEM) images of the *Haematococcus pluvialis* cell added with fungi shown in Fig. 17 in the induction stage where culture was performed while inducing biomineralization. Crystallization of the fungi by biomineralization was observed (Fig. 19) and complete transformation of the fungi into calcium carbonate was observed (Fig. 20). In addition, calcium carbonate was separated from the *Haematococcus pluvialis* cell without any energy consumption (Fig. 20).

The fungi, brown algae, and bacteria were biomineralized and observed with a scanning electron microscope (SEM). Fig. 20 shows scanning electron microscopy (SEM) images showing the progress of biomineralization induced by the fungi, Fig. 21 shows scanning electron microscopy (SEM) images showing the progress of biomineralization induced by the brown algae, and Fig. 22 shows scanning electron microscopy (SEM) images showing the progress of biomineralization induced by the bacteria. The images of Figs. 20 to 22 revealed the occurrence of biomineralization by the fungi, other microalgae (brown algae), and bacteria, indicating that cells other than the *Haematococcus pluvialis* cells were removed and separated.

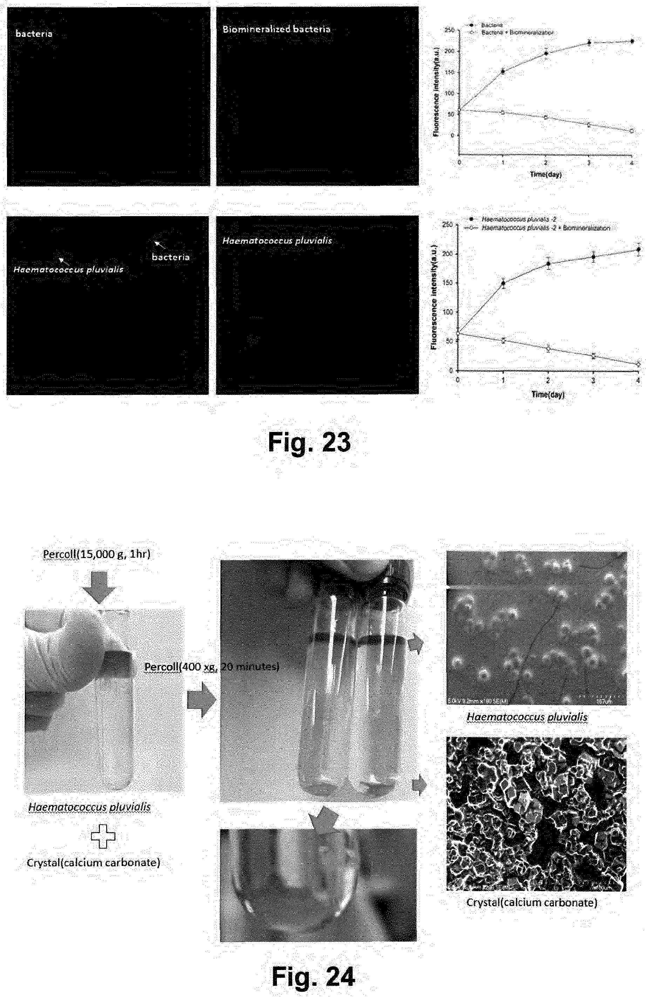

Fig. 23 shows microscopy (SEM) images and fluorescence intensities showing the progress of biomineralization induced by bacteria. Bacteria were stained and observed with a microscope during culture with and without adding calcium chloride to investigate the occurrence of biomineralization by the bacteria. As a result, the bacteria were continuously observed in the absence of calcium chloride but were not observed by biomineralization in the presence of calcium chloride. When the bacteria are cultured without mineralization, the fluorescence intensity continued to increase after cell staining. In contrast, when mineralization occurred, the fluorescence intensity converges to zero (0), demonstrating the death of the bacteria. When mineralization occurred in the presence of the bacteria and *Haematococcus pluvialis,* the fluorescence intensity of the bacteria converged to zero.

### Experimental Example 5: Separation of biomass and calcium carbonate

Fig. 24 shows a process for separating *Haematococcus pluvialis* biomass and precipitated calcium carbonate using Percoll and scanning electron microscopy (SEM) images of the separated biomass and calcium carbonate. Referring to Fig. 24, 8 ml of Percoll was injected into a glass vial, centrifuged for 1 h, injected with calcium carbonate-containing *Haematococcus pluvialis,* and centrifuged. The *Haematococcus pluvialis* having a relatively low density and the precipitated calcium carbonate having a relatively high density were separated into a supernatant and a lower layer.

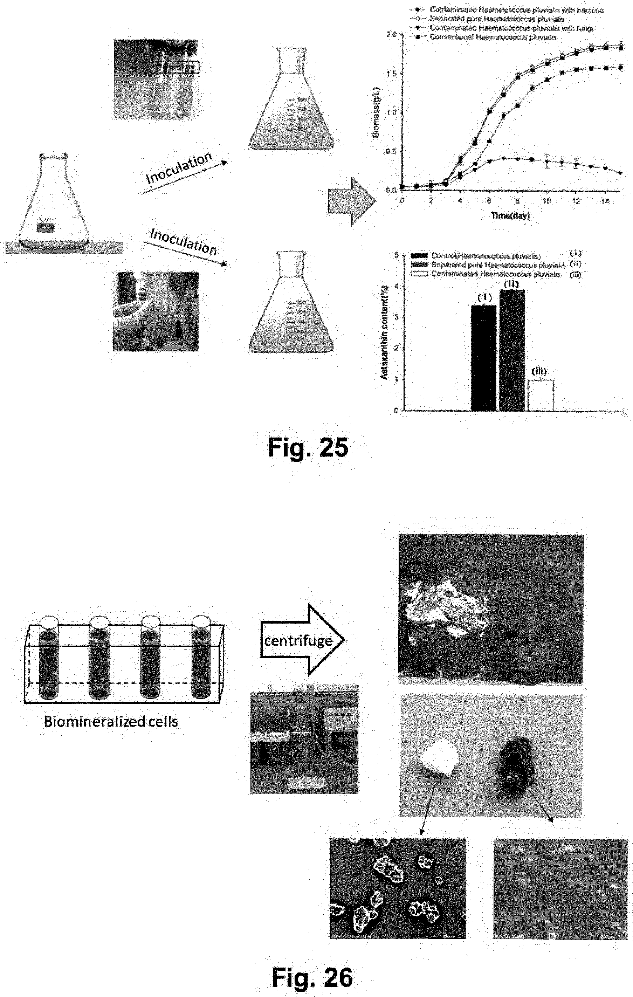

The separated *Haematococcus pluvialis* was cultivated under autotrophic conditions for 15 days and the yield of biomass produced was measured. The results are shown in Fig. 25. Fig. 25 shows graphs showing the yield (g/L) of biomass and the content (%) of astaxanthin after the separated pure *Haematococcus pluvialis* shown in Fig. 24 was cultivated under autotrophic conditions for 15 days. For comparison, conventional *Haematococcus pluvialis* was cultivated in the absence of sodium acetate and calcium chloride under autotrophic conditions, contaminated *Haematococcus pluvialis* with bacteria was cultivated under the same conditions, and contaminated *Haematococcus pluvialis* with fungi was cultivated under the same conditions. The yields of biomass produced were measured. As a result, when the *Haematococcus pluvialis* separated using Percoll was re-cultivated, the highest yield of biomass and the highest content of astaxanthin were obtained.

The separation mode using Percoll is suitable for a small amount of biomass and a continuous type centrifuge can be used to separate a large amount of biomass. Fig. 26 shows a process for separating *Haematococcus pluvialis* biomass and precipitated calcium carbonate using a large-capacity centrifuge and scanning electron microscopy (SEM) images of the separated biomass and calcium carbonate. Referring to Fig. 26, *Haematococcus pluvialis* biomass containing calcium carbonate produced in the graduated cylinders was separated using a large-capacity centrifuge. The biomass could be easily separated from the calcium carbonate crystal despite the presence of water therein.

Although the present invention has been described herein with reference to the specific embodiments, these embodiments do not serve to limit the invention and are set forth for illustrative purposes.

## Claims

1. A method for producing *Haematococcus pluvialis,* comprising (a) supplementing a medium with carbon dioxide to form bicarbonate ions (HCO₃⁻), (b) inoculating *Haematococcus pluvialis* into the medium supplemented with the carbon dioxide, (c) adding sodium acetate to the medium inoculated with the *Haematococcus pluvialis,* followed by first photo-culture, and (d) adding calcium ions (Ca²⁺) to the medium added with the sodium acetate to form calcium carbonate (CaCO₃), followed by second photo-culture,
wherein, in step (d), the calcium ions are added at a concentration of 8 to 18 mM,
wherein step (d) is carried out when the density of the *Haematococcus pluvialis* cells is 1.0 g/L or more in step (c),
wherein, in step (c), the sodium acetate is added at a concentration of 5 to 15 mM, and
wherein the zeta potential of the *Haematococcus pluvialis* is from -30 to -34 eV in step (c) and from -6 to -10 eV in step (d).

2. The method according to claim 1, wherein, in step (d), the calcium ions are added by supplementing the medium with calcium chloride (CaCl₂).

3. The method according to claim 1, wherein at least one microorganism selected from bacteria, fungi, and microalgae other than *Haematococcus pluvialis* is generated in at least one of steps (a) to (c) and is killed by encapsulation with the calcium carbonate.

4. The method according to claim 1, wherein the calcium carbonate formed on the surface of the *Haematococcus pluvialis* cells induces an increase in the size of the *Haematococcus pluvialis* and is separated from the surface of the *Haematococcus pluvialis* cells with increased size.

5. The method according to claim 1, wherein, in step (d), a basic solution is added to the medium to maintain the pH of the medium at 7.5 to 8.5.

6. The method according to claim 1, further comprising separating the cultivated *Haematococcus pluvialis* and the calcium carbonate after step (d).

## Patentansprüche

1. Verfahren zur Herstellung von *Haematococcus pluvialis,* umfassend (a) Versetzen eines Mediums mit Kohlendioxid, um Bicarbonat-Ionen (HCO₃⁻ ) zu erzeugen, (b) Inokulieren *von Haematococcus pluvialis* in das mit Kohlendioxid versetzte Medium, (c) Zugeben von Natriumacetat zu dem mit dem *Haematococcus pluvialis* inokulierten Medium, gefolgt von einer ersten Photokultivierung, und (d) Zugeben von Calciumionen (Ca²⁺ ) zu dem mit Natriumacetat ergänzten Medium, um Calciumcarbonat (CaCO₃ ) zu erzeugen, gefolgt von einer zweiten Photokultivierung,
wobei in Schritt (d) die Calciumionen in einer Konzentration von 8 bis 18 mM zugegeben werden,
wobei Schritt (d) durchgeführt wird, wenn die Dichte der *Haematococcus pluvialis-*Zellen in Schritt (c) 1,0 g/l oder mehr beträgt,
wobei in Schritt (c) das Natriumacetat in einer Konzentration von 5 bis 15 mM zugegeben wird und
wobei das Zeta-Potential von *Haematococcus pluvialis* in Schritt (c) zwischen -30 und -34 eV und in Schritt (d) zwischen -6 und -10 eV liegt.

2. Verfahren nach Anspruch 1, wobei in Schritt (d) die Calciumionen zugegeben werden, indem das Medium mit Calciumchlorid (CaCl₂) versetzt wird.

3. Verfahren nach Anspruch 1, wobei mindestens ein Mikroorganismus, ausgewählt aus Bakterien, Pilzen und Mikroalgen, die nicht *Haematococcus pluvialis* sind, in mindestens einem der Schritte (a) bis (c) erzeugt und durch Einkapselung mit dem Calciumcarbonat abgetötet wird.

4. Verfahren nach Anspruch 1, wobei das auf der Fläche der *Haematococcus* pluvialis-Zellen gebildete Calciumcarbonat eine Vergrößerung der *Haematococcus pluvialis* induziert und mit vergrößerter Größe von der Oberfläche der *Haematococcus* pluvialis-Zellen getrennt wird.

5. Verfahren nach Anspruch 1, wobei in Schritt (d) eine basische Lösung zu dem Medium zugegeben wird, um den pH-Wert des Mediums bei 7,5 bis 8,5 zu halten.

6. Verfahren nach Anspruch 1, das ferner das Trennen des kultivierten *Haematococcus pluvialis* und des Calciumcarbonats nach Schritt (d) umfasst.

## Revendications

1. Procédé de production d'*Haematococcus pluvialis,* comprenant (a) supplémenter un milieu en dioxyde de carbone pour former des ions bicarbonate (HCO₃⁻), (b) inoculer *Haematococcus pluvialis* dans le milieu supplémenté en dioxyde de carbone, (c) ajouter acétate de sodium au milieu inoculé avec *Haematococcus pluvialis,* suivi d'une première photo-culture, et (d) ajouter des ions de calcium (Ca²⁺) au milieu dans lequel de l'acétate de sodium a été ajouté pour former du carbonate de calcium (CaCO₃), suivi d'une deuxième photo-culture,
dans l'étape (d), les ions de calcium étant ajoutés à une concentration de 8 à 18 mM,
l'étape (d) étant réalisée lorsque la densité des cellules d'*Haematococcus pluvialis* est égale ou supérieure à 1.0 g/L dans l'étape (c),
dans l'étape (c), l'acétate de sodium étant ajouté à une concentration de 5 à 15 mM, et
le potentiel zêta de l'*Haematococcus pluvialis* étant dans la plage de -30 à -34 eV dans l'étape (c) et de -6 à -10 eV dans l'étape (d).

2. Procédé selon la revendication 1, dans lequel, dans l'étape (d), les ions de calcium sont ajoutés en supplémentant le milieu en chlorure de calcium (CaCl₂).

3. Procédé selon la revendication 1, dans lequel au moins un microorganisme choisi parmi bactéries, fungi, et microalgues autre que l'*Haematococcus pluvialis* est généré dans au moins une des étapes (a) à (c) et est tué par encapsulation avec du carbonate de calcium.

4. Procédé selon la revendication 1, dans lequel le carbonate de calcium formé sur la surface des cellules d'*Haematococcus pluvialis* induit une augmentation de la taille de l'*Haematococcus pluvialis* et est séparé de la surface des cellules d'*Haematococcus* pluvialis avec la taille accrue.

5. Procédé selon la revendication 1, dans lequel, dans l'étape (d), une solution basique est ajoutée au milieu pour maintenir le pH du milieu entre 7,5 et 8,5.

6. Procédé selon la revendication 1, comprenant en outre de séparer l'*Haematococcus pluvialis* cultivé et le carbonate de calcium après l'étape (d).
